# EUROPEAN PATENT APPLICATION

(11) **EP 2 047 847 A1**
(43) Date of publication of application: **15.04.2009**
(21) Application number: 07020034.0
(22) Date of filing: 12.10.2007
(51) Int. Cl.: A61K 31/18, A61K 9/32, A61K 9/36, A61K 9/16, A61K 9/20, A61K 9/26

(54) **Solid pharmaceutical composition comprising tamsulosin**

(71) Applicant: KRKA, tovarna zdravil, d.d., Novo mesto, 8501 Novo mesto (SI)
(72) Inventor: Sedmak, Gregor, 1000 Ljubljana (SI); Kramar, Andrejka, 8000 Novo mesto (SI); Vrecer, Franc, 8351 Straza pri Novem mestu (SI); Skrabanja, Vida, 8000 Novo mesto (SI)
(74) Representative: UEXKÜLL & STOLBERG

(57) **Abstract**

The present invention relates to a stable pharmaceutical composition comprising tamsulosin or a pharmaceutically acceptable salt or solvate thereof in a solid dosage form and to a process for preparing such a composition.

## Description

The present invention relates to a stable pharmaceutical composition comprising tamsulosin or a pharmaceutically acceptable salt or solvate thereof in a solid dosage form and to a process for preparing such a composition.

Tamsulosin is the generic name of 5-[(2R)-2-[[2-(2-Ethoxyphenoxy)ethyl]amino]propyl]-2-methoxybenzenesulfonamide and is known as an α₁-adrenergic receptor antagonist. For many years, tamsulosin or a salt thereof and in particular its hydrochloride salt has been used in the treatment of benign prostatic hyperplasia (BPH). BPH itself may not cause any symptoms but frequently leads to benign prostatic enlargement which can cause bladder outlet obstruction and may be associated with bothersome lower urinary tract symptoms (LUTS).

Pharmaceutical formulations of tamsulosin are available in the form of a modified-release (MR) capsule formulation or an orally-controlled absorption system (OCAS^{®}) tablet formulation.

For example, EP-A-0 661 045 is directed to adsorption system tablets and particularly describes sustained-release hydrogel preparations comprising (1) at least one drug, (2) an additive ensuring penetration of water into the preparation and (3) a hydrogel-forming polymer. The additive (2) is such that the amount of water required to dissolve 1 g of said additive is not more than 5 ml at a temperature of 20 ± 5°C. Due to the use of said additive the preparation undergoes a substantially complete gelation in the upper part of the GI tract, namely stomach and small intestine. The formed gel matrix is then maintained in the hydrated state in the colon and thus a sufficient drug release in the colon is achieved although water is poorly available there. This results in a uniform, sustained-release of the drug throughout the entire GI tract. In order that the release of the drug in the colon does not become insufficient, the amount of said additive (2) may not be too small and EP-A-0 661 045 teaches that the amount of said additive is generally about 5 to 80 wt.-%, based on the total weight of the preparation. Moreover, the amount of the hydrogel-forming polymer is said to be at least 70 mg and preferably at least 100 mg per preparation in order that the preparation will tolerate erosion in the digestive tract for a sufficiently long time and a sufficient sustained release will be achieved.

EP-A-1 568 361 relates to sustained-release compositions of tamsulosin having a specific drug release profile. In particular, the release of tamsulosin after 7 hours from the start of the test according to Japanese Pharmacopeia Dissolution Test Method 2 is about 20 to about 85%. The sustained-release compositions can be in the form of a hydrogel-forming preparation, a preparation of an osmotic pump type, a gel preparation having combined a plurality of gums, a multi-layered tablet comprising a geometrically aligned drug layer and release-controlling layer(s), a gastroretentive dosage form using a swelling polymer or a matrix preparation using water-soluble polymers.

EP-A-1 600 157 describes enteric sustained-release fine particles of tamsulosin that disintegrate in the buccal cavity and comprise (1) tamsulosin or a salt thereof, (2) an enterosoluble substance and when necessary (3) a water-insoluble polymer or wax which is appropriate for coating fine particles. The particles have a diameter of 5 to 250 µm and show a specific dissolution profile of tamsulosin.

However, there is still a need for a stable pharmaceutical composition of tamsulosin or a salt or solvate thereof having a satisfactory drug release profile which should not require the use of expensive excipients and should be obtainable by a simple and quick process. Moreover, dosage forms of tamsulosin having a smaller total weight than the known compositions would be desirable.

These objects are surprisingly solved by the solid pharmaceutical composition according to claim 1 to 17 or claim 18 to 26. The invention also relates to the process according to claim 27.

In a first aspect, the invention relates to a solid pharmaceutical composition which comprises
(a) at least one drug selected from tamsulosin or a pharmaceutically acceptable salt or solvate thereof,
(b) at least one matrix former and
(c) at least one disintegrant having a water-solubility of less than 0.2 g/ml at a temperature of 20°C,
wherein at least a part of said drug (a) and at least a part of said disintegrant (c) are dispersed in said matrix former (b).

The component (a) of the composition is a drug selected from tamsulosin or a pharmaceutically acceptable salt or solvate thereof. Examples of useful salts are the addition salts with an inorganic or organic acid. Examples of useful inorganic acids include hydrochloric, hydrobromic, phosphoric and sulfuric acid. Suitable organic acids can be selected from methanesulfonic, acetic, citric, tartaric, maleic, and glucoheptanoic acid. Examples of suitable solvents for preparing solvates of tamsulosin include water, ethanol and isopropanol. Preferably, the drug (a) is tamsulosin hydrochloride.

The drug (a) can be in a non-crystalline, i.e. amorphous, form or a crystalline form including any polymorphic or pseudopolymorphic forms such as solvates like hydrates. Preferably, a crystalline form of the drug and most preferably crystalline tamsulosin hydrochloride is used.

It is preferred that the composition comprises 0.02 to 5 wt.-%, more preferably 0.04 to 1 wt.-% and most preferably 0.08 to 0.8 wt.-% of drug (a).

It is also preferred that a single dosage form of the pharmaceutical composition according to the invention, such as a tablet, comprises 0.05 to 5 mg, preferably 0.1 to 1 mg and most preferably 0.2 and 0.8 mg of drug (a).

The component (b) of the composition according to the invention is at least one matrix former. Preferably, the matrix former (b) is selected from the group consisting of linear, branched and cross-linked polymers. Examples of a linear matrix forming polymeric material are polyethylene glycol and polyethylene oxide. Examples of a branched matrix forming polymeric material include cellulose derivatives such as hydroxypropyl methylcellulose (HPMC, hypromellose), carboxymethylcellulose, hydroxypropyl cellulose, methylcellulose and ethylcellulose, cellulose acetate, polyacrylates and polymethacrylates. Examples of a cross-linked matrix forming polymeric material is carbomer and polycarbophil. Natural hydrophilic polymers or theirs derivatives such as carrageenans, hitosan, xanthan, locus bean gum, gelan, hydroxypropyl starch, derivatives of alginic acid, dried molasses, guar gum, gum acacia, dextran, karaya gum, konjac gel, pectin, pulluan, scleroglucan, xyloglucan can also be used as matrix formers of the composition according to the present invention.

Preferably, the matrix former (b) can be selected from the group consisting of polyethylene glycol, polyethylene oxide, cellulose derivatives such as hydroxypropyl methylcellulose, carboxymethylcellulose, hydroxypropyl cellulose, methylcellulose and ethylcellulose., cellulose acetate, polyacrylates, polymethacrylates, carbomer, polycarbophil, carrageenans, hitosan, xanthan, locus bean gum, gelan, hydroxypropyl starch, derivatives of alginic acid, dried molasses, guar gum, gum acacia, dextran, karaya gum, konjac gel, pectin, pulluan, scleroglucan, xyloglucan and mixtures thereof.

Preferably, branched or cross-linked matrix formers are used. Moreover, it is particularly preferred that the matrix former (b) is selected from the group consisting of hydroxypropy methylcellulose, carbomer and mixtures thereof. Most preferably, a mixture of hydroxypropyl methylcellulose and carbomer is used as matrix former (b).

The composition preferably comprises 5 to 95 wt.-%, more preferably 10 to 90 wt.-% and most preferably 20 to 80 wt.-% of matrix former (b).

In particular, 5 to 95 wt.-%, more preferably 10 to 90 wt.-% and most preferably 20 to 80 wt.-% of hydroxypropyl methylcellulose and/or 0.01 to 20 wt.-%, more preferably 0.05 to 10 wt.-% and most preferably 0.1 to 5 wt.-% of carbomer is used as matrix former (b). If a mixture of hydroxypropyl methylcellulose and carbomer is used as matrix former (b), the weight ratio of hydroxypropyl methylcellulose to carbomer preferably ranges from 200:1 to 3:1, more preferably 150:1 to 5:1 and most preferably 40:1 to 20:1 or 140:1 to 120:1.

The composition also comprises at least one disintegrant (c) which has a water-solubility of less than 0.2 g/ml at a temperature of 20°C. Preferably, disintegrant (c) has a water-solubility of less than 0.1 g/ml, more preferably less than 0.05 g/ml and most preferably less than 0.04 g/ml at a temperature of 20°C.

In a preferred embodiment, disintegrant (c) is practically insoluble in water at a temperature between 15°C and 25°C according to the definition in Pharm. Eur. 5.08

Suitable disintegrants (c) are selected from the group consisting of crospovidone, sodium starch glycolate, microcrystalline cellulose, cross-linked carboxymethylcellulose sodium (CMC-Na), polacrilin potassium and mixtures thereof. Preferably, the disintegrant is selected from the group consisting of crospovidone, cross-linked CMC-Na and mixtures thereof. Most preferably, crospovidone is used as disintegrant (c).

Preferably, the composition according to the invention comprises 0.1 to 30 wt.-%, more preferably 0.5 to 20 wt.-% and most preferably 1 to 10 wt.-% of disintegrant (c).

At least a part of components (a) and (c) are dispersed in the matrix former (b). Preferably, the majority of components (a) and (c) and more preferably all of the components (a) and (c) are dispersed in the matrix former (b). Thus, the drug (a) is in contact with the matrix former (b) and the disintegrant (c) without being separated in different layers. Preferably, components (a) to (c) are mixed together alone or optionally with at least one other excipient to form a homogeneous mixture,
wherein particles of drug (a) are in contact with particles of components (b) and (c) and optionally at least one other excipient. It is preferred that the composition according to the invention is not in a form of multi-layered tablets comprising components (a) to (c) in different layers. Moreover, it is preferred that the composition is not in a form of osmotic pump type preparations. In fact, the composition according to the invention is preferably in the form of a matrix preparation such as a matrix tablet. As used herein, the term "matrix preparation" refers to preparations as for example described in J.R. Cardinal, Drug release from matrix devices, in: J.M. Anderson, S.W. Kim (Eds.), Recent Advances in Drug Delivery Systems, Plenum Press, New York, 1984, pp. 229-248.

It has surprisingly been found that by selecting the above components a stable pharmaceutical composition showing a suitable dissolution profile of tamsulosin can be prepared by using a very simple and fast process. In particular, the compositions do not require use of an additive ensuring penetration of water into the preparation which has such a solubility that the amount of water for dissolving 1 g of the additive is not more than 5 ml at a temperature of 20°C. Therefore, the composition according to the invention preferably comprises less than 10 wt.-%, more preferably less than 5 wt.-% such as 0 to 4 wt.-% of such an additive. It is particularly preferred that the compositions according to the invention does not comprise any such additive.

Furthermore, it has surprisingly been found that the amount of hydrogel-forming polymers used in the compositions according to the invention can be lower than in the formulations known in the prior art. It is particularly preferred that a single dosage form of the composition according to the invention comprises less than 100 mg and most preferably less than 70 mg of a hydrogel-forming polymer. As used herein, the term "hydrogel-forming polymer" refers to polymers as defined.and illustrated in EP-A-0 661 045.

In addition to components (a) to (c), the composition according to the invention can comprises at least one other excipient selected from the group consisting of diluents, binders, lubricants, surfactants, stabilizing agents and mixtures thereof.

Examples of diluents useful in the composition according to the present invention include, but are not restricted to pharmaceutically acceptable substances which are insoluble or only partially soluble in water, i.e. having a water-solubilty of less than 0.2 g/ml at room temperature, such as diluents selected from the group consisting of microcrystalline cellulose, powdered cellulose, lactose (anhydrous and monohydrate), siliconised microcrystalline cellulose, calcium hydrogen phosphate, calcium carbonate, calcium lactate and any mixtures thereof. Preferably, microcrystalline cellulose and/or lactose and more preferably microcrystalline cellulose can be used as diluent.

Examples of binders useful in the composition according to the present invention include, but are not restricted to polyvinylpyrrolidone, microcrystalline cellulose, hydroxyethyl cellulose, hydroxypropyl cellulose, hydroxypropyl methylcellulose or other cellulose ethers, starch, pregelatinized starch, polymethacrylates and/or any mixtures thereof. Preferred binders are those which have a solubility in water of less than 20 weight/vol-% at room temperature, such as microcrystalline cellulose.

The composition according to the invention can comprise a lubricant selected from, but not restricted to the group consisting of stearic acid, stearic acid salts, such as magnesium stearate, magnesium palmitate, and magnesium oleate, hydrogenated vegetable oil, hydrogenated castor oil, talc, sodium stearyl fumarate, macrogols and mixtures thereof. Preferably, the composition comprises at least one lubricant selected from stearic acid, magnesium stearate and hydrogenated vegetable oil.

Further, the composition according to the invention can comprises at least one surfactant selected from the group consisting of ionic surfactants such as sodium dodecyl sulfate and non-ionic surfactants such as sorbitane derivatives (e.g. polysorbate 80), sugar esters, poloxamers and mixtures thereof.

Although the composition according to the invention comprising components (a) to (c) shows a satisfactory stability, it can comprise at least one stabilizing agent which may be a substance having an antioxidizing effect such as iron oxides, butyl hydroxyltoluene, butyl hydroxyanizole, ascorbic acid, derivatives of flavonoids and isoflavonoids such as rutin, quercetin, gallic acid and coffee acid.

Optionally, the composition according to the invention comprises at least one coating. Preferably, the coating comprises a polymer selected from the group consisting of water .insoluble or soluble cellulose derivatives such as ethylcellulose, polyvinyl acetate, methacrylic ester copolymers, ammonio methacrylate copolymers, aminoalkyl methacrylate copolymers, methacrylic acid copolymers, polyvinyl alcohol, methylcellulose, hydroxypropyl methylcellulose and mixtures thereof.

Further, the coating can comprise at least one other excipient selected from the group consisting of plasticizers, antitacking agents, release rate modifiers, colorants, opacifiers, pigments, vehicles and mixtures thereof.

Preferably, the composition according to the invention is in the form of cores or tablets which can be coated with an appropriate material used for film coating, e.g. as described in Pharmaceutical Coating Technology (G. Cole (ed.), 1995). Film coating formulations usually contain components like polymer(s), plasticizer(s), colorant(s)/opacifier(s), vehicle(s). In film coating suspension the minor quantities of flavors, surfactants and waxes can be used. Film coating suspensions can also be used as ready-to-make preparations which are available on the market.

In a specific embodiment of the present invention, a tablet matrix system can be coated by a controlled release film coating comprising controlled release polymer which can be selected from water insoluble cellulose derivatives such as ethylcellulose, polyvinyl acetate, methacrylic ester copolymers (Eudragit NE) and ammonio methacrylate copolymers. Controlled release coating can further contain plasticizers which lower the glass transition temperature of controlled release polymers below 80°C, preferably below 60°C, antitacking agents such as talc, glycerol monostearate, release rate modifiers which can be selected from pharmaceutically acceptable substances having solubility in water at least 10 weight/vol-% such as mannitol, sorbitol, lactose, polyethylene glycol 1500, polyethylene glycol 6000, polyethylene glycol 20000, povidone, low viscosity cellulose ether derivatives such as hyprollose, hypromellose, hydroxyethyl cellulose, having a viscosity of 2% solution in water at room temperature 1 to 30 cp, preferably 2 to 15 cp, more preferably 3 to 9 cp determined according to the method described in US Pharmacopoeia, colorants and pigments such as titanium dioxide, iron oxides.

In another embodiment of present invention, a tablet matrix system can be coated by a film coating having low permeability for water vapour and/or oxygen which comprises polymers such as aminoalkyl methacrylate copolymers (Eudragit E types), methacrylic acid copolymers, polyvinyl alcohol, methylcellulose, hypromellose, plasticizers such as polyethylene glycol having a molecular weight below 10,000, propylene glycol triethylcitrate, antitacking agents such as talc, glycerol monostearate, colorants and pigments such as titanium dioxide, iron oxides.

According to a preferred embodiment, the composition according to the invention comprises
(a) tamsulosin hydrochloride,
(b) at least one matrix former,
(c) crospovidone and
(d) optionally microcrystalline cellulose.

According to a particularly preferred embodiment, the composition according to the invention comprises
(a) tamsulosin hydrochloride,
(b) hydroxypropyl methylcellulose and/or carbomer,
(c) crospovidone and
(d) optionally microcrystalline cellulose.

It is particularly preferred that the composition according to invention comprises, based on the total weight of the composition,
(a) 0.08 to 0.8 wt.-% of tamsulosin hydrochloride,
(b') 20 to 80 wt.-% of hydroxypropyl methylcellulose, (b'') 0.1 to 5 wt.-% of carbomer,
(c) 1 to 10 wt.-% of crospovidone and
(d) 0 to 70.wt.-%, preferably 20 to 60 wt.-% of microcrystalline cellulose.

According to the second aspect, the invention relates to a solid pharmaceutical composition which comprises
(a) at least one drug selected from tamsulosin or a pharmaceutically acceptable salt or solvate thereof,
(b) at least one matrix former,
(c) at least one disintegrant having a water-solubility of less than 0.2 g/ml at a temperature of 20°C and
(d) optionally at least one other excipient,
   wherein said drug (a), at least a part of said matrix former (b) and optionally at least one other excipient (d) such as a diluent and/or a binder form an agglomerate.

Preferably, said agglomerate is present in admixture with said disintegrant (c) and optionally at least one other excipient which can be same or different from excipient (d).

According to this aspect, the composition according to the invention comprises agglomerates. These agglomerates can be obtained by granulation and are thus also referred to as granulates. Granulation can be performed by a wet or dry process,
wherein wet granulation using water or organic solvents or mixtures thereof as granulation liquid and dry granulation can be performed by processes known as slugging and/or roller compaction. Preferably, the average particle size of the agglomerates/granulates measured by laser diffraction (Malveren) ranges from 50 to 500µm, more preferably 80 to 400µm and even more preferably 100 to 300µm. The agglomerates/granulates comprise said drug (a), at least a part of said matrix former (b) and optionally at least one other excipient (d) and preferably are present in admixture with said disintegrant (c) and optionally at least one other excipient which can be the same or different from excipient (d).

All preferred embodiments described above for the composition according to the first aspect of the invention are also preferred embodiments of the composition according to the second aspect of the invention. This for example means that also the composition according to the second aspect of the invention most preferably comprises crystalline tamsulosin hydrochloride as drug (a). Likewise, all preferred embodiments described for the composition according to the first aspect of the invention in claims 2 to 17 below are also preferred embodiments of the composition according to the second aspect of the invention.

The invention also relates to a process for preparing the composition according to the invention comprising
(i) converting components (a) to (c) and optionally other excipients into a particulate form and
(ii) optionally coating the composition obtained in step (i).

In step (i) components (a) to (c) and optionally other excipients are converted into a particulate form. This can be done by any known process such as wet or dry granulation, direct compression methods etc. It is preferred that step (i) is performed by firstly granulating drug (a), at least a part of the matrix former (b) and optionally at least one other excipient and then mixing the obtained agglomarates or granulates with disintegrant (c) or optionally other excipients. Once the composition has been prepared, it can be compression-molded into tablets with a diameter from 1.5 to 12 mm, preferably 2 to 10 mm, more preferably 4 to 8 mm and weight 40 to 800 mg, preferably 50 to 600 mg and more preferably 80 to 400. mg. The shape of tablets can be of any known form, oblong and round shape being preferred in order to alleviate the swallowing of the tablet by patients having problems with swallowing, especially geriatric patients.

In a special embodiment of present invention tamsulosin or its pharmaceutically acceptable salts or solvates can be formulated into matrix spherical particles with controlled release of drug, which can be filled into capsules or sachets or compressed into tablets.

In step (ii) the composition obtained in step (i) can be coated with a coating material. Tablet coating equipment known from the state of the art can be used for this step. Water, organic solvents such as ethanol, acetone, isopropanole or mixtures thereof can be used for preparing coating dispersions.

The invention is further illustrated by reference to the following examples which are not intended to limit the scope of the invention.

### Examples

### Examples 1 to 5 - Preparation of compositions of tamsulosin hydrochloride

| Example | 1 | 2 | 3 | 4 | 5 |
|---|---|---|---|---|---|
| | mass (mg) | mass (mg) | mass (mg) | mass (mg) | mass (mg) |
| Tamsulosin hydrochloride | 0.4 | 0.4 | 0.4 | 0.4 | 0.4 |
| Hypromellose (Methocel K100M) | 154.9 | 63.0 | 137.8 | 57.0 | 63.0 |
| Microcrystalline cellulose (Avicel PH 101) | 82.7 | 29.0 | 82.7 | 29.0 | 26.5 |
| Carbomer (Garbopol 71G) | 5.0 | 2.0 | 20.0 | 8.0 | 2.0 |
| Crospovidone (Kollidon CL) | 3.0 | 4.0 | 5.0 | 4.0 | 4.0 |
| Silica (Aerosil 200) | 1.5 | 0.6 | 1.5 | 0.6 | 0.6 |
| Magnesium stearate | 2.5 | 1.0 | 2.5 | 1.0 | 1.0 |
| D-Sorbitol | - | - | - | - | 2.5 |

Tamsulosin hydrochloride, microcrystalline cellulose and a part of Methocel K100M were granulated using a mixture of ethanol and water. After the granulation step, the remaining components were added and mixed to obtain a homogeneous mixture suitable for tableting. Tablets were prepared on a rotary press.

### Examples 6 to 13 - Preparation of coated compositions of tamsulosin hydrochloride

| Examples | 6 | 7 | 8 | 9 | 10 | 11 | 12 | 13 |
|---|---|---|---|---|---|---|---|---|
| | mass (mg) | mass (mg) | mass (mg) | mass (mg) | mass (mg) | mass (mg) | mass (mg) | mass (mg) |
| Tamsulosin hydrochloride | 0.4 | 0.4 | 0.4 | 0.4 | 0.4 | 0.4 | 0.4 | 0.4 |
| Hypromellose (Methocel K100M) | 64.5 | 63.0 | 64.5 | 63.0 | 64.5 | 63.0 | 64.5 | 63.0 |
| Microcrystalline cellulose (Avicel PH 101) | 29.0 | 29.0 | 23.0 | 23.0 | 89.0 | 89.0 | 83.0 | 83.0 |
| Carbomer (Carbopol 971P) | 0.5 | 2.0 | 0.5 | 2.0 | 0.5 | 2.0 | 0.5 | 2.0 |
| Crospovidone (Kollidon CL) | 4.0 | 4.0 | 10.0 | 10.0 | 4.0 | 4.0 | 10.0 | 10.0 |
| Silica (Aerosil 200) | 0.6 | 0.6 | 0.6 | 0.6 | 0.6 | 0.6 | 0.6 | 0.6 |
| Magnesium stearate | 1.0 | 1.0 | 1.0 | 1.0 | 1.0 | 1.0 | 1.0 | 1.0 |

Tamsulosin hydrochloride, microcrystalline cellulose and a part of Methocel K100M were granulated using a mixture of ethanol and water. After the granulation step, the remaining components were added and mixed to obtain a homogeneous mixture suitable for tableting. Tablets were prepared on a rotary press. The obtained tablet cores were further coated with a coating water suspension containing ethylcellulose, low molecular weight.hypromellose having a viscosity of less than 30 cp of a 2% solution in water at room temperature and talc. The coating was performed in a drum of an ordinary coating machine.

### Example 14 - Preparation of a composition of tamsulosin hydrochloride by direct compression

The components of the composition according to Example 6 were mixed together and compressed into tablets.

### Example 15 - Preparation of a composition of tamsulosin hydrochloride via dry granulation

| | mass (mg) |
|---|---|
| Tamsulosin hydrochloride | 0.4 |
| Hypromellose (Methocel K100M) | 64.5 |
| Microcrystalline cellulose (Avicel PH 101) | 29.0 |
| Carbomer (Carbopol 971P) | 0.5 |
| Crospovidone (Kollidon CL) | 4.0 |
| Silica (Aerosil 200) | 0.6 |
| Magnesium stearate | 1.0 |

The drug, a part of hypromellose and microcrystalline cellulose were compacted on a roller compactor. The obtained compactat was milled and sieved to obtain a granulate. The obtained granulate was mixed with the remaining components and compressed into tablets.

### Example 16 - Preparation of a coated composition of tamsulosin hydrochloride

The tablet cores prepared according to Example 5 were coated with a dispersion which was prepared from (composition is given for 5,000 tablet cores):

| | |
|---|---|
| Ehylcellulose (Surelease E-7_19040) | 15.00 g |
| Hypromellose 6cp | 0.75 g |
| Talc | 1.25 g |
| Purified water | 24.25.g |

The hypromelose was dissolved in purified water and mixed with the Surelease dispersion. Talc is dispersed in the mixture and the obtained suspension is sprayed onto tablet cores using a laboratory Manesty tablet coater (perforated drum).

## Claims

1. Solid pharmaceutical composition comprising
(a) at least one drug selected from tamsulosin or a pharmaceutically acceptable salt or solvate thereof,
(b) at least one matrix former and
(c) at least one disintegrant having a water-solubility of less than 0.2 g/ml at a temperature of 20°C,
wherein at least a part of said drug (a) and at least a part of said disintegrant (c) are dispersed in said matrix former (b).

2. Composition according to claim 1, wherein the drug (a) is tamsulosin hydrochloride.

3. Composition according to claim 1 or 2, wherein the matrix former (b) is selected from the group consisting of linear, branched and cross-linked polymers.

4. Composition according to claim 3, wherein the matrix former (b) is selected from the group consisting of polyethylene glycol, polyethylene oxide, cellulose derivatives such as hydroxypropyl methylcellulose, carboxymethylcellulose, hydroxypropyl cellulose, methylcellulose and ethylcellulose, cellulose acetate, polyacrylates, polymethacrylates, carbomer, polycarbophil, carrageenans, hitosan, xanthan, locus bean gum, gelan, hydroxypropyl starch, derivatives of alginic acid, dried molasses, guar gum, gum acacia, dextran, karaya gum, konjac gel, pectin, pulluan, scleroglucan, xyloglucan and mixtures thereof.

5. Composition according to claim 4, wherein the matrix former (b) is selected from the group consisting of hydroxypropyl methylcellulose, carbomer and mixtures thereof.

6. Composition according to any one of claims 1 to 5, wherein the disintegrant (c) has a water-solubility of less than 0.1 g/ml at a temperature of 20°C.

7. Composition according to claim 6, wherein the disintegrant (c) is practically insoluble in water at a temperature between 15°C and 25°C.

8. Composition according to any one of claims 1 to 7, wherein the disintegrant (c) is selected from the group consisting of crospovidone, sodium starch glycolate, microcrystalline cellulose, cross-linked carboxymethylcellulose sodium (CMC-Na), polacrilin potassium and mixtures thereof.

9. Composition according to claim 8, wherein the disintegrant (c) is selected from the group consisting of crospovidone, cross-linked CMC-Na and mixtures thereof.

10. Composition according to claim 9, wherein the disintegrant (c) is crospovidone.

11. Composition according to any one of claims 1 to 10, which further comprises at least one other excipient selected from the group consisting of diluents, binders, lubricants, surfactants, stabilizing agents and mixtures thereof.

12. Composition according to any one of claims 1 to 11, which further comprises at least one coating.

13. Composition according to claim 12, wherein the coating comprises a polymer selected from the group consisting of water insoluble or soluble cellulose derivatives, such as ethylcellulose, polyvinyl acetate, methacrylic ester copolymers, ammonio methacrylate copolymers, aminoalkyl methacrylate copolymers, methacrylic acid copolymers, polyvinyl alcohol, methylcellulose, hydroxypropyl methylcellulose and mixtures thereof.

14. Composition according to claim 12 or to 13, wherein the coating further comprises at least one other excipient selected from the group consisting of plasticizers, antitacking agents, release rate modifiers, colorants, opacifiers, pigments, vehicles and mixtures thereof.

15. Composition according to any one of claims 1 to 14, which comprises
(a) tamsulosin hydrochloride,
(b) at least one matrix former,
(c) crospovidone and
(d) optionally microcrystalline cellulose.

16. Composition according to claim 15, which comprises
(a) tamsulosin hydrochloride,
(b) hydroxypropyl methylcellulose and/or carbomer,
(c) crospovidone and
(d) optionally microcrystalline cellulose.

17. Composition according to claim 16, which comprises, based on the total weight of the composition,
(a) 0.08 to 0.8 wt.-% of tamsulosin hydrochloride,
(b') 20 to 80 wt.-% of hydroxypropyl methylcellulose,
(b'') 0.1 to 5 wt.-% of carbomer,
(c) 1 to 10 wt.-% of crospovidone and
(d) 0 to 70 wt.-% of microcrystalline cellulose.

18. Solid pharmaceutical composition comprising
(a) at least one drug selected from tamsulosin or a pharmaceutically acceptable salt or solvate thereof.,
(b) at least one matrix former,
(c) at least one disintegrant having a water-solubility of less than 0.2 g/ml at a temperature of 20°C and
(d) optionally at least one other excipient,
wherein said drug (a), at least a part of said matrix former (b) and optionally at least one other excipient (d) form an agglomerate.

19. Composition according to claim 18, wherein said agglomerate is present in admixture with said disintegrant (c) and optionally at least one other excipient which can be the same or different from excipient (d).

20. Composition according to claim 18 or 19, wherein the drug (a) is defined as in claim 2.

21. Composition according to any one of claims 18 to 20,
wherein the matrix former (b) is defined as in any one of claims 3 to 5.

22. Composition according to any one of claims 18 to 21,
wherein the disintegrant (c) is defined as in any one of claims 6 to 10.

23. Composition according to any one of claims 18 to 22,
wherein the excipient (d) is defined as in claim 11.

24. Composition according to any one of claims 18 to 23,
wherein the composition comprises at least one coating.

25. Composition according to claim 24, wherein the coating is defined as in claim 13 or 14.

26. Composition according to any one of claims 18 to 25, which comprises components (a) to (d) as defined in any one of claims 15 to 17.

27. Process for preparing the composition according to any one of claims 1 to 19 comprising
(i) converting components (a) to (c) and optionally other excipients into a particulate form and
(ii) optionally coating the composition obtained in step (i).
